(19) Europäisches Patentamt
European Patent Office
Office européen des brevets

(11) **EP 2 638 851 A1**

(12) **EUROPEAN PATENT APPLICATION**

(43) Date of publication:
**18.09.2013 Bulletin 2013/38**

(51) Int Cl.:
**A61B 5/00** *(2006.01)*    **G01N 21/17** *(2006.01)*

(21) Application number: **13000839.4**

(22) Date of filing: **19.02.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **13.03.2012 JP 2012056014**

(71) Applicant: **Canon Kabushiki Kaisha Tokyo (JP)**

(72) Inventor: **Oishi, Takuji Tokyo (JP)**

(74) Representative: **Weser, Wolfgang Weser & Kollegen Patentanwälte Radeckestrasse 43 81245 München (DE)**

(54) **Photoacoustic imaging facilitating distinction between optical absorber images and artifacts**

(57)     A subject information obtaining device according to the present disclosure includes: first image data obtaining means (252) arranged to obtain first image data based on a first detection signal obtained by detecting first photoacoustic waves generated by irradiating light in a first measurement state on a subject (130, 131); second image data obtaining means (252) arranged to obtain second image data based on a second detection signal obtained by detecting second photoacoustic waves generated by irradiating light in a second measurement state different from the first measurement state on the subject (130, 131); and image data output means (253) arranged to display the first image data and the second image data on display means (280) so as to compare the first image data and the second image data at the display means (280).

FIG. 3

**Description**

BACKGROUND OF THE INVENTION

Field of the Invention

One disclosed aspect of the embodiments relates to a subject information obtaining device, a subject information obtaining method, and a program which obtain subject information by detecting photoacoustic waves generated by light being irradiated.

Description of the Related Art

With the medical field, in recent years, photoacoustic imaging (PAI) whereby living body function information is obtained using light and ultrasonic waves has been proposed as one of devices capable of imaging the interior of a living body noninvasively, and its development has advanced.

The photoacoustic imaging mentioned here is technology to irradiate pulsed light generated from a light source upon a subject, and image internal tissue serving as a generation source of photoacoustic waves, using photoacoustic advantages that photoacoustic waves (typically, ultrasonic waves) are generated by absorption of light spread and diffused within the subject. Specifically, signals obtained by detecting change resulting from time of received photoacoustic waves at multiple locations are mathematically analyzed, i.e., restructured, and information relating to an optical characteristic value within a subject is visualized three-dimensionally.

In the event of employing near-infrared light as pulsed light, near-infrared light has a property to readily penetrate water making up a majority portion of a living body and to readily be absorbed by hemoglobin in blood, which enables a blood vessel image to be imaged. Further, oxygen saturation in blood which is function information may be measured by comparing blood vessel images obtained from pulsed light beams with different wavelengths. It has been thought that blood around a malignant tumor is lower in oxygen saturation than blood around a benign tumor, and accordingly, there is anticipation that malignant/benign judgment of a tumor may be performed by knowing oxygen saturation.

Japanese Patent Laid-Open No. 2010-35806 has disclosed that a concentration distribution of a substance making up a living body is imaged by the photoacoustic imaging.

However, as disclosed in Japanese Patent Laid- Open No. 2010- 35806, with the photoacoustic imaging, at the time of imaging photoacoustic waves, artifacts emerging in a position where there is actually no optical absorber hinder observation of optical absorbers.

For example, in the event that a subject is held at a holding plate of which the acoustic impedance differs from a subject, photoacoustic waves generated within the subject are reflected multiply within the holding plate.

At the time of detecting photoacoustic waves thus reflected multiply for imaging, artifacts due to multiple reflections emerge. Such artifacts hinder an optical absorber image actually existing from being distinguished.

Therefore, it has been found to be desirable to provide a subject information obtaining device and a subject information obtaining method which facilitate distinction between optical absorber images and artifacts.

SUMMARY OF THE INVENTION

The present invention in its first aspect provides a subject information obtaining device as specified in claims 1 to 15.

The present invention in its second aspect provides a subject information obtaining method as specified in claims 16 to 17.

The present invention in its third aspect provides a program as specified in claim 18.

Further features of the present invention will become apparent from the following description of exemplary embodiments with reference to the attached drawings.

BRIEF DESCRIPTION OF THE DRAWINGS

Figs. 1A to 1D are diagrams for describing principles of the present disclosure.

Fig. 2 is a diagram illustrating a configuration of a subject information obtaining device according to a first embodiment.

Fig. 3 is a diagram illustrating a flowchart for a subject information obtaining method according to the first embodiment.

Figs. 4A and 4B are diagrams illustrating an example of a first measurement state and a second measurement state according to the first embodiment.

Figs. 5A and 5B are diagrams illustrating another example of the first measurement state and second measurement state according to the first embodiment.

Figs. 6A and 6B are diagrams illustrating another example of the first measurement state and second measurement state according to the first embodiment.

Figs. 7A and 7B are diagrams illustrating another example of the first measurement state and second measurement state according to the first embodiment.

Figs. 8A and 8B are diagrams illustrating another example of the first measurement state and second measurement state according to the first embodiment.

Fig. 9 is a diagram illustrating an example of a display device where image data is displayed, according to the first embodiment.

Fig. 10 is a diagram illustrating another example of the display device where image data is displayed, according to the first embodiment.

Fig. 11 is a diagram illustrating a flowchart for a subject information obtaining method according to a second embodiment.

Fig. 12 is a diagram illustrating an example of a display device where image data and a light intensity distribution are displayed, according to the second embodiment.

Fig. 13 is a diagram illustrating another example of the display device where image data and a light intensity distribution are displayed, according to the second embodiment.

Fig. 14 is a diagram illustrating another example of the display device where image data and a light intensity distribution are displayed, according to the second embodiment.

Fig. 15 is a diagram illustrating a flowchart for a subject information obtaining method according to a third embodiment.

Fig. 16 is a diagram illustrating an example of a display device where image data and confidence regions are displayed, according to the third embodiment.

DESCRIPTION OF THE EMBODIMENTS

Artifacts are observed in the same way if measurement is performed in the same way since artifacts are high in reproducibility. Therefore, artifacts emerge in the same way with measurement, and accordingly, an optical absorber image and artifacts are not readily distinguished.

With the present disclosure, with the photoacoustic imaging, multiple measurements with different measurement states are performed and results thereof are displayed so as to be compared. Thus, optical absorber images and artifacts may be distinguished.

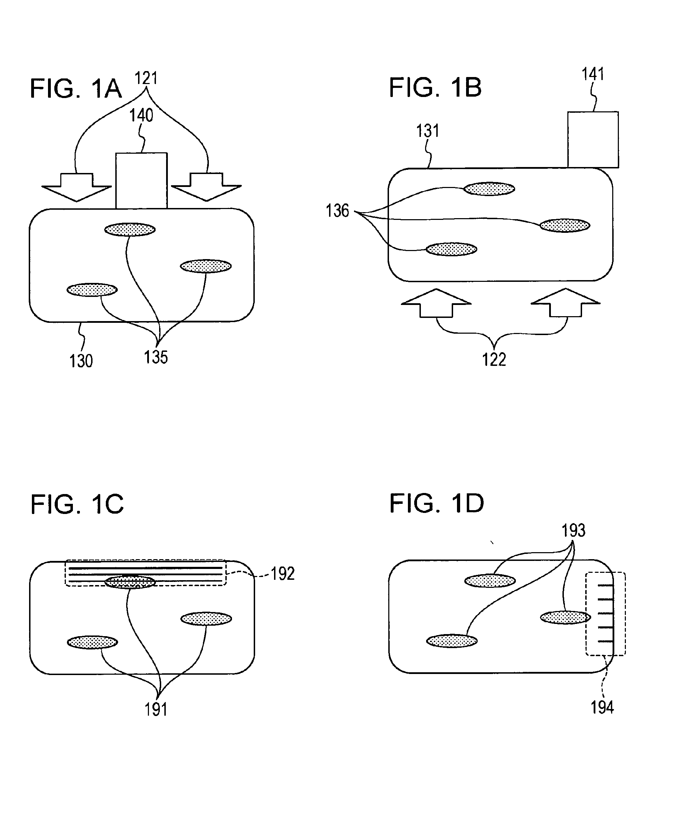

First, principles of the present disclosure will be described with reference to Figs. 1A to 1E. Figs. 1A to 1E illustrate an image of a position relation (measurement state) between irradiation light and an acoustic wave detector and a subject, and an initial sound pressure distribution obtained at the time of this position relation. Here, subjects 130 and 131 include optical absorbers 135 and 136 in the same positions, respectively.

With a measurement state illustrated in Fig. 1A, light 121 in a first measurement state is irradiated on a surface of the subject 130 acoustically connected to a detection surface of an acoustic wave detector 140.

Also, with a measurement state illustrated in Fig. 1B, light 122 in a second measurement state is irradiated on a surface of the subject 131 facing an acoustic wave detector 141. Also, in Fig. 1B, a portion of the acoustic wave detector 141 is not acoustically connected to the subject 131.

Further, an initial sound pressure distribution obtained at the time of the measurement state in Fig. 1A is illustrated in Fig. 1C. Also, an initial sound pressure distribution obtained at the time of the measurement state in Fig. 1B is illustrated in Fig. 1D.

It is understandable that appearing positions of artifacts 192 and 194 differ depending on measurement states when comparing Figs. 1C and 1D. On the other hand, it is understandable that appearing positions of optical absorber images 191 and 193 are the same even when changing the measurement state. This is because a process for occurrence of artifacts differs depending on measurement states. Hereinafter, the process for occurrence of artifacts in each of the measurement states will be described.

With the measurement state in Fig. 1A, the light 121 in the first measurement state also partially generates wraparound photoacoustic waves on the surface of the acoustic wave detector 140. The photoacoustic waves generated on the surface of the acoustic wave detector 140 then appear as the artifacts 192 due to multiple reflections or the like in an acoustic matching layer of the acoustic wave detector 140 or the like.

Also, with the measurement state in Fig. 1B, of the acoustic wave detector 141, the light 122 in the second measurement state wrapped around the subject 131 directly inputs to a portion which is not acoustically connected to the subject 131 to generate photoacoustic waves on the surface of the acoustic wave detector 141. The photoacoustic waves then appear as the artifacts 194 due to multiple reflections or the like in the acoustic matching layer of the acoustic wave detector 141.

In this manner, the optical absorber images and artifacts may be distinguished by comparing Figs. 1C and 1D which are initial sound pressure distributions when their measurement states differ.

As described above, there may be provided a subject information obtaining device which facilitates optical absorber images and artifacts to be distinguished by displaying image data obtained in the multiple measurement states so as to compare the image data.

Note that, with the present disclosure, the image data means data to display information relating to optical characteristic values on a display unit: Also, the optical characteristic values include initial sound pressure and optical energy density obtained by performing reconstitution processing on detection signals, or something relating to optical absorption coefficients obtained by performing light intensity correction thereon.

Hereinafter, embodiments of the present disclosure will be described.

First Embodiment

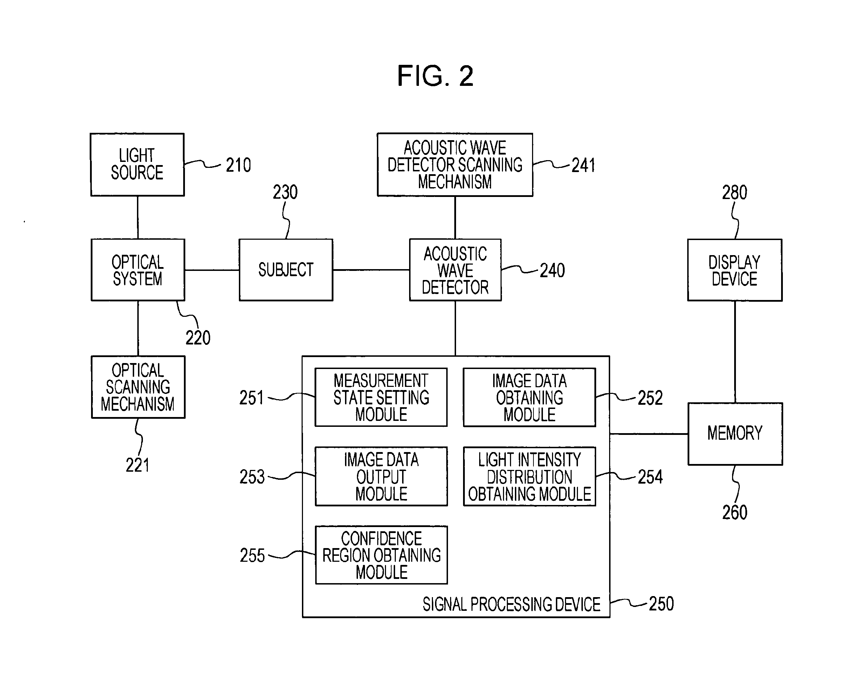

Fig. 2 is a block diagram illustrating a configuration of a subject information obtaining device according to the present embodiment, and is configured of a light source 210, an optical system 220, an optical scanning mechanism 221, a subject 230, an acoustic wave detector 240, an acoustic wave detector scanning mechanism 241, a signal processing device 250, memory 260, and a display device 280. The signal processing device 250 according to the present embodiment includes a measurement state setting module 251 serving as a measurement state setting unit, an image data obtaining module 252 serving as an image data obtaining unit, an image data output module 253 serving as an image data output unit, a light intensity distribution obtaining module 254 serving as a light intensity distribution obtaining unit, and a confidence region obtaining module 255 serving as a confidence region obtaining means (255).

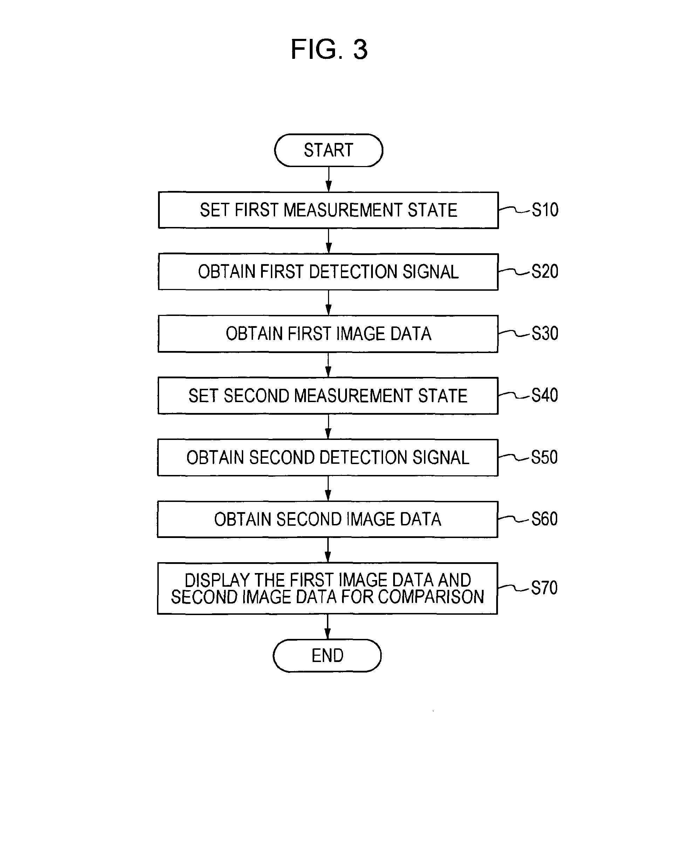

Fig. 3 is a diagram illustrating a flow of a subject information obtaining method using the subject information obtaining device according to the present embodiment illustrated in Fig. 2.

First, the measurement state setting module 251 controls the light source 210, optical system 220, and acoustic wave detector 240 to set these in the first measurement state, and light in the first measurement state is irradiated on the subject 230 (S10). The acoustic wave detector 240 then detects first photoacoustic waves generated at the subject 230 by the light in the first measurement state to obtain a first detection signal (S20).

Next, the image data obtaining module 252 serving as a first image data obtaining unit performs reconstitution processing using this first detection signal, thereby obtaining a first initial sound pressure distribution serving as first image data, and saving this in the memory 260 (S30).

Next, the measurement state setting module 251 sets the light source 210, optical system 220, and acoustic wave detector 240 in the second measurement state, and light in the second measurement state is irradiated on the subject 230 (S40). The acoustic wave detector 240 then detects second photoacoustic waves generated at the subject 230 using the light in the second measurement state to obtain a second detection signal (S50).

Next, the image data obtaining module 252 serving as a second image data obtaining unit performs reconstitution processing using this second detection signal, thereby obtaining a second initial sound pressure distribution serving as second image data, and saving this in the memory 260 (S60).

Next, the image data output module 253 outputs the first image data and second image data saved in the memory 260 so as to compare the first image data and second image data at the display device 280 (S70).

The first image data and second image data are then displayed on the display device 280 so as to compare the first image data and second image data output to the display device 280.

Note that the image data obtaining module 252 may obtain an initial sound pressure distribution by performing reconstitution processing using a heretofore known reconstituting method such as a universal back-projection method to overlap signals subjected to differential processing, for example, as described in (Minghua Xu and Lihong V. Wang, (2005), "Universal back-projection algorithm for photoacoustic computed tomography", PHYSICAL REVIEW E 71, 016706), or the like.

Also, a program including the above-mentioned processes may be executed by the signal processing device 250 serving as a computer.

S10, S40: Processes to Set Measurement State

Next, setting for a measurement state illustrated in S10 and S40 in Fig. 3 will be described in detail.

Hereinafter, description will be made regarding an example wherein the measurement state setting module 251 sets a measurement state. The measurement states in the present disclosure are conception including an irradiated state (irradiated position, irradiated angle, and irradiated intensity) of irradiation light and the detection position of an acoustic wave detector.

First, description will be made regarding an example wherein the irradiated state of irradiation light serving as a measurement state is changed.

1. Example for Irradiating Light in Different Positions of Subject Surface

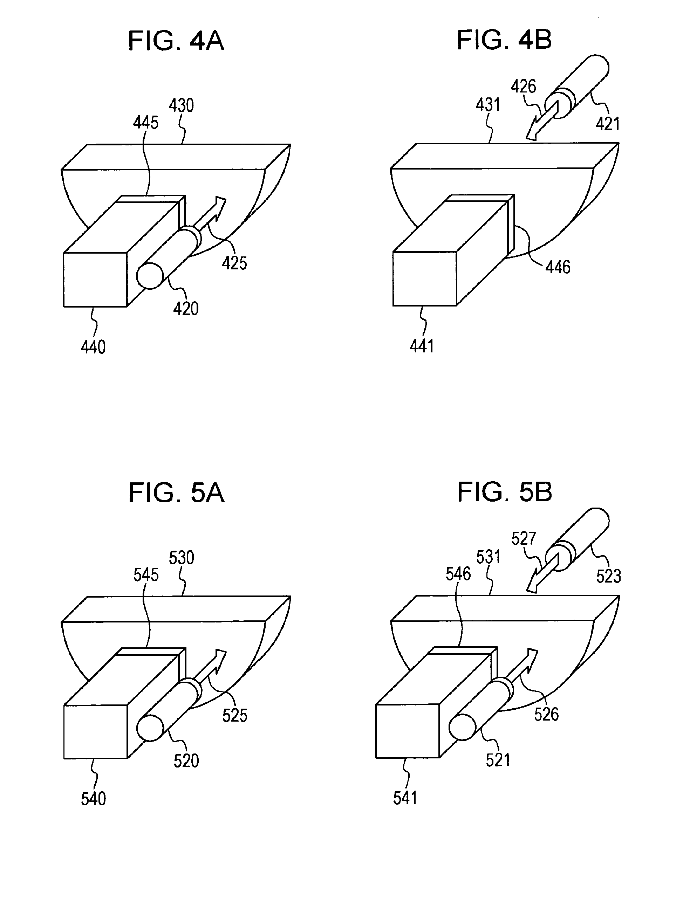

First, description will be made regarding an example of a measurement state in which light in a first measurement state and light in a second measurement state are irradiated on different positions of a subject surface, with reference to Figs. 4A, 4B, 5A, and 5B.

With a first measurement state illustrated in Fig. 4A, light 425 in the first measurement state is irradiated on a surface of a subject 430 acoustically connected to a detection surface 445 of an acoustic wave detector 440 from an optical system 420. Next, with a second measurement state illustrated in Fig. 4B, light 426 in the second measurement state is irradiated on a surface facing a surface of a subject 431 acoustically connected to a detection surface 446 of an acoustic wave detector 441 from an optical system 421.

In this manner, with each of the measurement states, in the event that light is irradiated on different positions of a subject surface, and each of the measurement results is taken as image data, processes for occurrence of artifacts differ, and accordingly, artifacts emerge in different positions. Therefore, image data obtained by irradiating light on different positions of a subject surface is displayed so as to be compared, and accordingly, an optical absorber image and artifacts may be distinguished.

Also, even with the measurement states illustrated in Figs. 5A and 5B, the present disclosure may be applied. The first measurement state illustrated in Fig. 5A is the same as the measurement state illustrated in Fig. 4A. On the other hand, with the second measurement state illustrated in Fig. 5B, light 525 in the same measurement state as the measurement state illustrated in Fig. 4A, and light 526 in the same measurement state as the measurement state illustrated in Fig. 4B are irradiated on the surfaces of a subject 531.

In this manner, even in the event that the light in the second measurement state includes not only the light in the first measurement state but also the light irradiated on a position of the subject surface different from the light in the first measurement state, positions and intensities of artifacts occurring differ, and accordingly, optical absorber images and artifacts may be distinguished.

Note that irradiated position for the subject surface may be changed by changing a beam profile of light in the subject surface in each measurement state.

Also, the greater a position to be irradiated of the subject surface is changed depending on measurement states, the greater an occurrence position of artifacts is also changed, and accordingly, it is desirable to change an irradiated position depending on measurement states.

2. Example for Irradiating Light with Different Angle Made Up of Subject Surface and Irradiation Direction

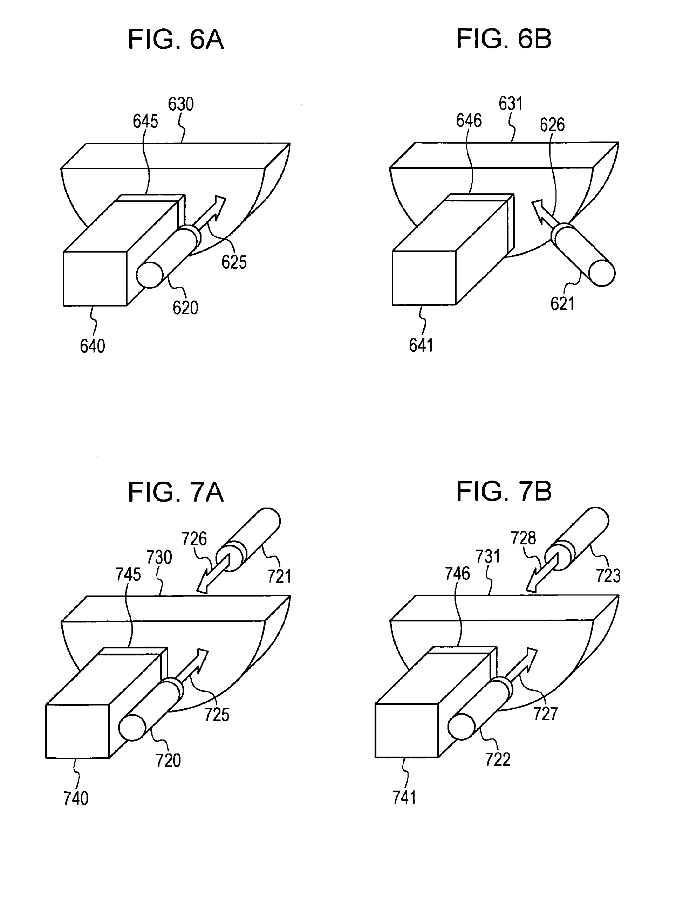

Next, description will be made regarding an example of a measurement state with a different irradiation angle which is an angle made up of a subject surface and the irradiation direction of light, with reference to Figs. 6A and 6B.

A first measurement state illustrated in Fig. 6A is the same as the measurement state illustrated in Fig. 4A. On the other hand, with a second measurement state illustrated in Fig. 6B, light 626 in the second measurement state is irradiated on the same position as an irradiation position of light 625 in the first measurement state illustrated in Fig. 6A with a irradiation angle different from the light 625 in the first measurement state from an optical system 621.

In this case as well, when comparing image data obtained in the measurement state illustrated in Fig. 6A, and image data obtained in the measurement state illustrated in Fig. 6A, positions where artifacts emerge differ.

Therefore, image data obtained in multiple measurement states with a different angle made up of a subject surface and irradiation direction is displayed so as to be compared, and accordingly, optical absorber images and artifacts may be distinguished.

Note that the greater the irradiation angle is changed depending on measurement states, the greater an occurrence position of artifacts is also changed, and accordingly, it is desirable to greatly change the irradiation angle depending on measurement states.

3. Example for Irradiating Light with Different Irradiation Intensity

Next, description will be made regarding an example of a measurement state with different irradiation intensity of light in each measurement state, with reference to Figs. 7A and 7B.

With the measurement states illustrated in Figs. 7A and 7B, an optical system includes multiple light irradiating units, and multiple light beams are irradiated on different positions of a subject surface from each of the light irradiating units.

Here, light 725 in a first measurement state illustrated in Fig. 7A, and light 727 in a second measurement state illustrated in Fig. 7B are irradiated on the same position, and light 726 in a first measurement state illustrated in Fig. 7A, and light 728 in a second measurement state illustrated in Fig. 7B are irradiated on the same position.

With the measurement state illustrated in Fig. 7A, the light 725 with weak intensity is irradiated on a surface of a subject 730 acoustically connected to an acoustic wave detector 740, and the light 726 with strong intensity is irradiated on a surface of the subject 730 facing that surface.

On the other hand, with the measurement state illustrated in Fig. 7B, the light 727 with strong intensity is irradiated on a surface of a subject 731 acoustically connected to an acoustic wave detector 741, and the light 728 with weak intensity is irradiated on a surface of the subject 731 facing that surface.

When light is irradiated from the multiple light irradiating units, photoacoustic waves corresponding to each irradiation light beam from each light irradiating unit occur. Artifacts corresponding to the intensity of each irradiation light then emerge, and image data to which the artifacts thereof are added is obtained. Accordingly, a ratio between intensity of an optical absorber image and intensity of artifacts differs depending on measurement states, and accordingly, the optical absorber image and artifacts may be distinguished.

As a method to change irradiation intensity of light in each measurement state, there are conceived a method for providing a filter to attenuate light to an optical system, a method to adjust output of a light source corresponding to light in each measurement state, and so forth. Additionally, any method may be employed as long as the method enables to change irradiation intensity of light in each measurement state.

As described above, with the method to change the measurement state by changing multiple irradiation intensities, as compared to a method to change the irradiation position or irradiation angle, driving of the optical system may be reduced. In particular, in the event of adjusting output of the light source, driving of the optical system may considerably be reduced. Therefore, mechanical movement of the device may be reduced, and automation of measurement may simply be realized.

Note that, in the event that light is irradiated from the multiple light irradiating units, it is desirable to change irradiation intensity in each measurement state with the optical system fixed. Precision of the irradiation position may be improved by fixing the optical system.

With the present disclosure, that the irradiation intensity of light in the first measurement state and the irradiation intensity of light in the second measurement state differ indicates that the irradiation intensity of light in each measurement state simply differs in the event that there is one light emitting unit in the optical system. Also, in the event that there are multiple light emitting units in the optical system, this indicates that, of multiple light beams from the multiple light emitting units, the irradiation intensity of at least one light beam differs.

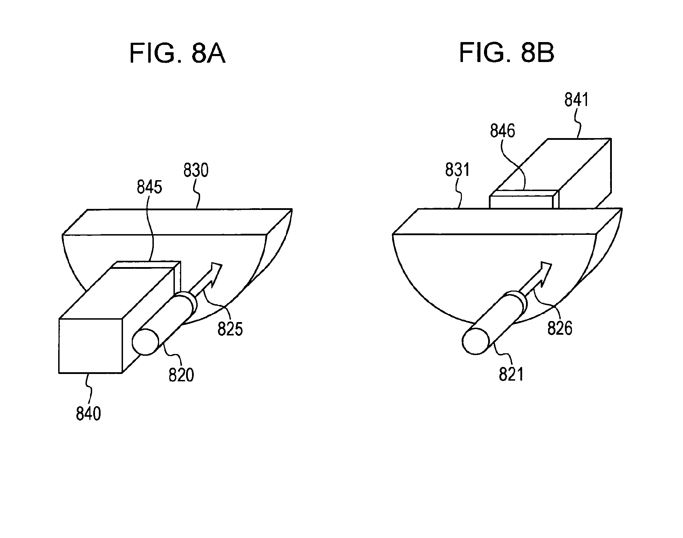

Next, description will be made regarding an example for changing the detection position of an acoustic wave detector serving as a measurement state, with reference to Figs. 8A and 8B.

4. Example for Detecting Photoacoustic Waves in Position Where Detection Surface of Acoustic Wave Detector Differs

A first measurement state illustrated in Fig. 8A is the same as the measurement state illustrated in Fig. 4A.

On the other hand, with a second measurement state illustrated in Fig. 8B, though the irradiation state of light 826 in the second measurement state is the same as the irradiation state of light 825 in the first measurement state, the position of a detection surface 846 of an acoustic wave detector 841 differs from the position of a detection surface 845 of an acoustic wave detector 840 in the first measurement state.

As illustrated in Figs. 8A and 8B, even in the event that the position of the detection surface of the acoustic wave detector differs depending on measurement states, artifacts emerge on a different position depending on measurement states.

This is because whether or not there are multiple reflections which occur by light being irradiated on the surface of an acoustic wave detector, or a propagation path of photoacoustic waves, and so forth depend on the position of the acoustic wave detector. Therefore, appearance positions of artifacts depend on the position of the detection surface of the acoustic wave detector. Accordingly, multiple image data obtained by changing the position of the detection surface of the acoustic wave detector depending on measurement states are displayed for comparison, and accordingly, optical absorber images and artifacts may be distinguished.

Note that, in the event of having changed an angle made up of the detection surface of the acoustic wave detector and a subject depending on measurement states as well, positions where artifacts appear differ depending on measurement states. In this case, the position of the detection surface of the acoustic wave detector is consequently changed depending on each of the measurement states by changing an angle made up of the detection surface of the acoustic wave detector and a subject surface. That is to say, with the present disclosure, that the position of the detection surface of the acoustic wave detector differs includes that an angle made up of the detection surface of the acoustic wave detector and a subject surface differs.

Note that, with processes in S10 and S40, in the event that light is irradiated on multiple positions, or in the event that light is irradiated from multiple light irradiating units, these light beams do not have to be irradiated at the same time. Specifically, it may be performed to irradiate serial light on each irradiation position, or to irradiate serial light from each irradiating unit.

Also, in the event of irradiating light on multiple different positions, it is desirable to scan the optical system using the optical scanning mechanism. Also, at this time, the optical system may be scanned by the measurement state setting module controlling the optical scanning mechanism.

Also, in the event that the detection surface of the acoustic wave detector detects photoacoustic waves in multiple positions, photoacoustic waves may be detected at multiple positions using multiple acoustic wave detectors, or an acoustic wave detector may be scanned by the acoustic wave detector scanning mechanism to detect photoacoustic waves at multiple positions. Also, at this time, the measurement state setting module may scan the acoustic wave detector by controlling the acoustic wave detector scanning mechanism.

Also, the optical system and acoustic wave detector may be scanned while maintaining a relative position relation between the optical system and the acoustic wave detector.

Also, with the present embodiment, though the measurement state setting module sets the first measurement state and second measurement state by controlling driving of the light source or optical system, a worker may set the light source or optical system so as to realize the first measurement state and second measurement state.

Also, at the time of comparing multiple measurement states, in the event that the number of indications of image data with a different measurement state increases, discriminating precision between optical absorber images and artifacts is enhanced, and accordingly, it is desirable to increase the number of measurement states.

S70: Process to Display First Image Data and Second Image Data for Comparison

Next, description will be made regarding an example of a method for displaying first image data and second image data illustrated in S70 in Fig. 3 so as to compare these.

Hereinafter, an example will be described wherein parallel display, superimposed display, and alternating display are performed on the display device.

1. Example for Displaying First Image Data and Second Image Data in Parallel

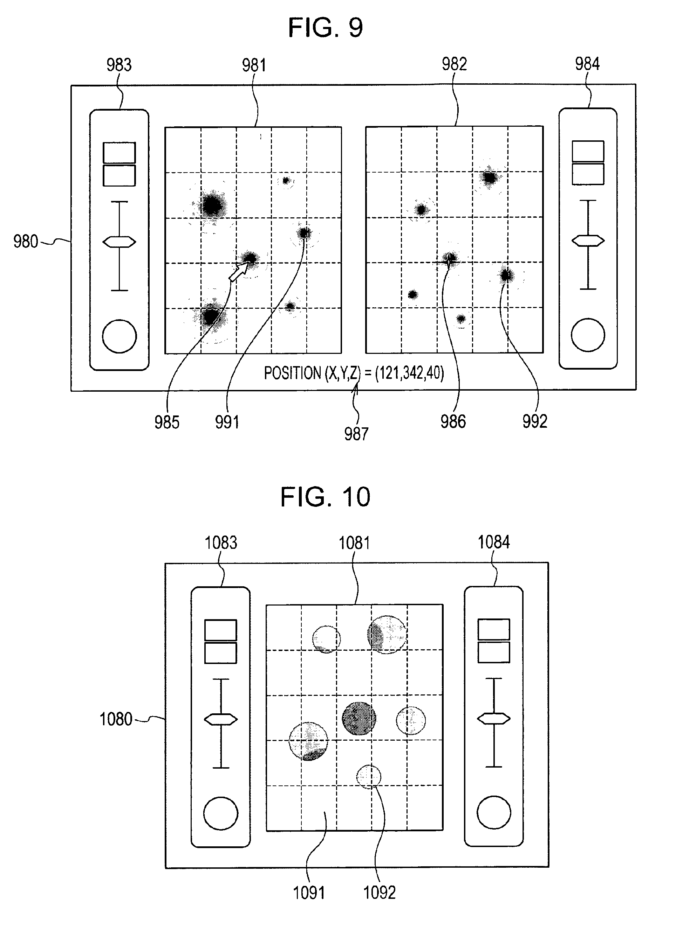

First, a method for displaying the first image data and second image data in a row will be described with reference to Fig. 9.

A display device 980 illustrated in Fig. 9 includes a first display region 981 and a second display region 982. The image data output module 253 outputs first image data 991 to the first display region 981, and outputs second image data 992 to the second display region 982.

As a result thereof, the first image data 991 is displayed on the first display region 981, and the second image data 992 is displayed on the second display region 982.

Also, the display device 980 includes a first operating unit 983 corresponding to the first display region 981, and a second operating unit 984 corresponding to the second display region 982. However, a single operating unit may be provided so as to correspond to the multiple display regions.

Also, with the display region 980, a first pointer 985 within the first display region 981 is moved using the first operating unit 983, whereby information 987 such as coordinates of a position specified by the first pointer 985, and a data value at the coordinates thereof may be displayed.

Also, in order to facilitate comparison between the image data displayed in the first display region 981 and the image data displayed in the second display region 982, for example, when specifying certain coordinates within the first display region 981 using the first pointer 985, a second pointer 986 is displayed on a position corresponding to the first pointer 985 within the second display region 982.

Also, though it is desirable to display the first display region 981 and second display region 982 in the same location, same range, and same dynamic range in an interlocking manner, the display regions may individually be adjusted.

Also, though it is desirable that the display regions and operating units are provided to a single display device, multiple display devices to which the display regions and operating units are provided may be prepared.

In this manner, image data in different measurement states are displayed in a row, and accordingly, each image data may independently be diagnosed, and consequently, visibility of each image data is good. Therefore, optical absorber images and artifacts may be distinguished.

2. Example for Displaying First Image Data and Second Image Data in Superimposed Manner

Next, an example of a method for displaying the first image data and second image data in a superimposed manner will be described with reference to Fig. 10.

A display device 1080 illustrated in Fig. 10 includes a single display region 1081. The image data output module 253 outputs first image data 1091 and second image data 1092 to the single display region 1081. As a result thereof, the first image data 1091 and second image data 1092 are displayed in the display region 1081 in a superimposed manner.

Also, the display device 1080 includes a first operating unit 1083 corresponding to the first image data 1091, and a second operating unit 1084 corresponding to the second image data 1092. Though, in order to handle the two image data, the two operating units are provided here, multiple image data may be handled by a single operating unit.

As illustrated in Fig. 10, in the event of displaying image data in a superimposed manner, each image data may be displayed by changing its transmittance so as to visibly recognize each image data even when multiple image data are overlapped.

Also, an arrangement may be made wherein a color is assigned to each image data, and intensity of image data is displayed with shading of its color. Also, an arrangement may be made wherein shading is assigned to each image data, and intensity of image data is represented with its color.

Also, it is desirable that a worker performs setting of transmittance, colors, shading, and so forth using an operating unit provided to the display device so as to perform the setting in an interactive manner.

Also, of multiple image data, only optional image data may be displayed in a superimposed manner. At this time, it is desirable that optional image data may be selected using an operating unit.

As described above, multiple image data are displayed in a superimposed manner, thereby facilitating comparison of the coordinates, shapes, sizes, and so forth of images, and facilitating distinction between optical absorber images and artifacts.

3. Example for Alternately Displaying First Image Data and Second Image Data

Next, an example of a method for displaying the first image data and second image data on the same coordinates in a temporally alternating manner will be described.

The image data output module 253 outputs each of multiple image data saved in the memory to a single display region of the display device at different timing. The multiple image data are displayed on the single display region by being switched at different timing.

At this time, though it is desirable that timing that the image data output module 253 outputs each image data is an operator's optional timing by operations at the operating unit, each image data may automatically be output by determining timing to output the image data beforehand. In the event of automatically outputting each image data, the image data that has been output first may be output after outputting the last image data.

As described above, each image data is alternately displayed on the single display region, thereby reducing visible hindrance due to overlapping of each image data, and facilitating distinction between optical absorber images and artifacts.

Note that, before a process to display image data for comparison illustrated in S70, the signal processing device may perform preprocessing such as blurring processing, enhancement processing, or the like on image data.

The blurring processing mentioned here is processing to blur image data, which removes high-frequency random noise, and also deteriorates sensitivity for misalignment between measurement states. Specifically, a Gaussian filter, a spatial-frequency low pass filter, a moving average filter, or the like may be employed as the blurring processing.

Also, the enhancement processing is processing for emphasizing a portion matched with a pattern peculiar to optical absorbers in image data. Specifically, the template matching method may be used for image data by taking a pattern peculiar to optical absorbers as a template. Also, the template matching method may be used by taking a pattern peculiar to artifacts or random noise as a template. At this time, there may be performed processing to increase or processing to decrease intensity of image data of a region extracted by the template matching method, or processing to increase or processing to decrease intensity of image data of a region other than a region extracted by the template matching method.

In this manner, the image data in each measurement state subjected to the preprocessing is image data where an optical absorber image or artifacts have been enhanced. Accordingly, image data is subjected to the preprocessing, whereby distinction between optical absorbers and artifacts may more readily be performed when comparing imaged data in each measurement state.

As described above, distinction between optical absorber images and artifacts may readily be facilitated by comparing image data obtained in the different measurement states.

Second Embodiment

The present embodiment differs from other embodiments in that a light intensity distribution within a subject in each measurement state is displayed in addition to image data obtained in each measurement state. Here, the light intensity distribution includes data to be displayed on the display device, obtained by performing luminance value conversion on the light intensity distribution.

Incidentally, initial sound pressure PO of photoacoustic waves depends on light intensity Φ as represented with a relation in Expression (1) .

$$P0 = \Gamma \cdot \Phi \cdot \mu a \quad . \quad . \quad . \quad (1)$$

Here, Γ represents a Grueneisen constant, and μa represents an optical absorption coefficient. As represented with Expression (1) , when light intensity irradiated on an optical absorber has a great value, initial sound pressure of photoacoustic waves to be generated also increases. Specifically, an SN ratio of a detection signal corresponding to a region where much light intensity is irradiated increases. Therefore, image data of a region where much light intensity is irradiated is image data obtained from a detection signal with a great SN ratio, and accordingly, reliability is high. On the contrary, reliability of image data of a region where light is insufficiently irradiated is low. Therefore, with the present embodiment, not only image data obtained in each measurement state but also a light intensity distribution within a subject in each measurement state are displayed, and accordingly, a region with high reliability may be determined with comparison of image data in each measurement state.

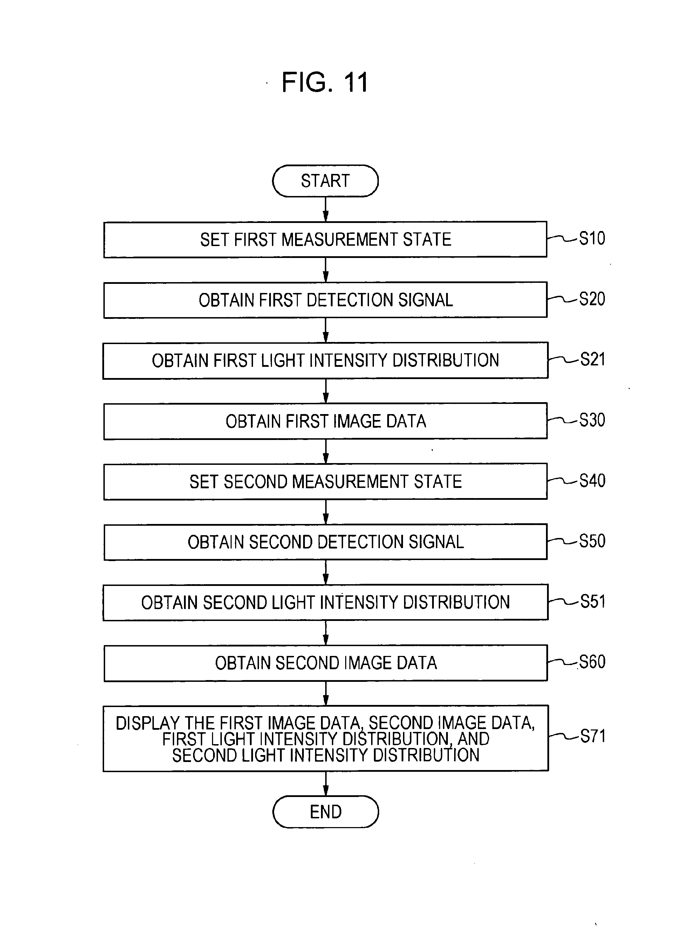

Hereinafter, a method for displaying a light intensity distribution will be described with reference to a flowchart illustrated in Fig. 11. Note that the same processing as with the flowchart illustrated in Fig. 3 will be denoted with the same processing number, and description thereof will be omitted. Also, description will be made using the subject information obtaining device illustrated in Fig. 2.

S21, S51: Process to Obtain Light Intensity Distribution

First, the light intensity distribution obtaining module 254 of the signal processing device 250 obtains a first light intensity distribution based on light in the first measurement state, and obtains a second light intensity distribution based on light in the second measurement state.

Here, as a method for obtaining a light intensity distribution, there may be employed a method for obtaining a light intensity distribution within a subject from a beam profile of irradiation light for the subject by calculation of light propagation within the subject. Also, an arrangement may be made wherein a beam profile of irradiation light for a subject, and a beam profile of light externally emitted from the subject are measured, and a light intensity distribution within the subject is obtained from a relation between both. Note that, in the event that the same beam profile of irradiation light for a subject is employed if irradiation settings are not changed, and accordingly, beam profile data saved in the memory beforehand may be employed instead.

S71: Process to Display Image Data and Light Intensity Distribution

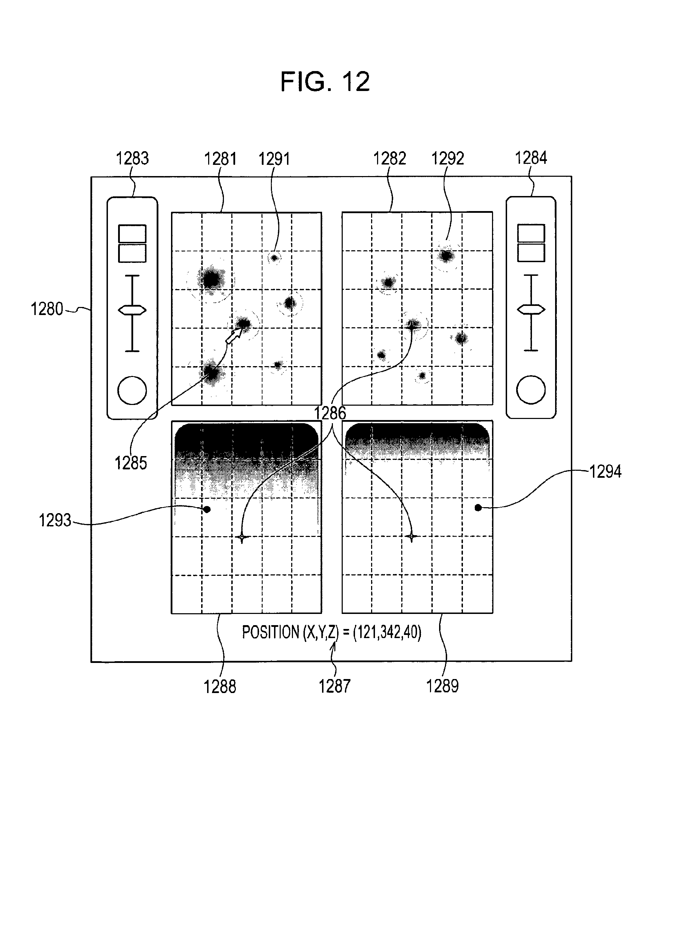

Next, the image data output module 253 outputs the first light intensity distribution, and second light intensity distribution to the display device 280, and causes the display device 280 to display the first light intensity distribution, and second light intensity distribution. Hereinafter, a display example of light intensity distributions will be described with reference to Figs. 12 to 14.

First, as illustrated in Fig. 12, an example will be described wherein image data and a light intensity distribution in the measurement states are output to separate display regions, and are displayed in a row, respectively.

First, the image data output module 253 outputs first image data 1291 to a first display region 1281, outputs second image data 1292 to a second display region 1282, outputs a first light intensity distribution 1293 to a third display region 1283, and outputs a second light intensity distribution 1294 to a fourth display region 1284. As illustrated in Fig. 12, each image data and each light intensity distribution are displayed on the display device 1280 in a row.

In this manner, the light intensity distributions are displayed in separate display regions, and accordingly, visibility for the light intensity distributions may be enhanced, respectively.

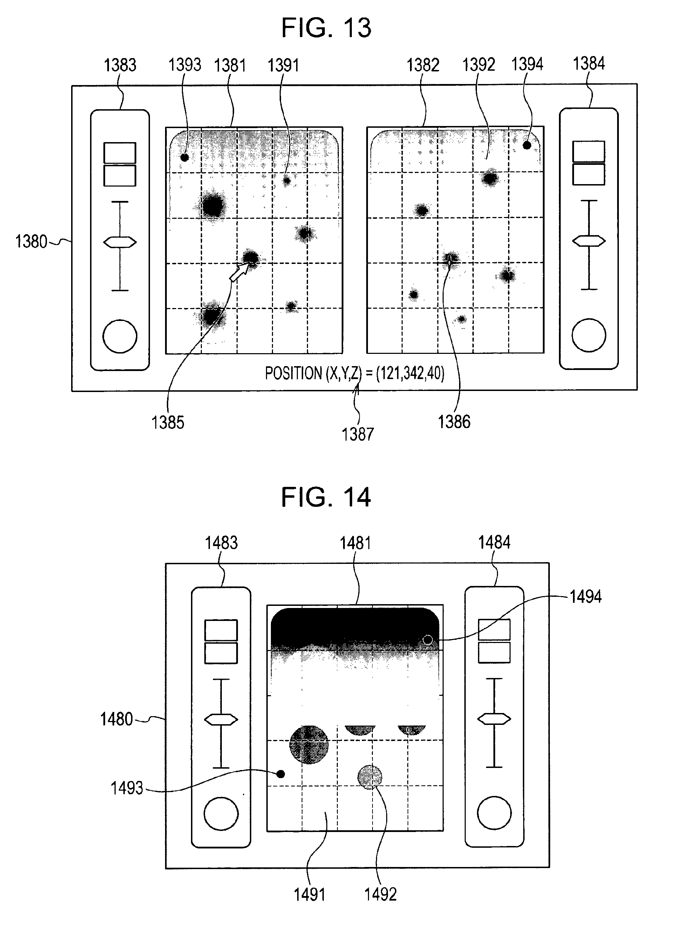

Next, as illustrated in Fig. 13, an example will be described wherein the light intensity distribution in each measurement state is displayed by being superimposed on image data corresponding to each of the light intensity distributions.

First, the image data output module 253 outputs first image data 1391 and a first light intensity distribution 1393 to a first display region 1381, and outputs second image data 1392 and a second light intensity distribution 1394 to a second display region 1382. As illustrated in Fig. 13, the first image data 1391 and first light intensity distribution 1393 are displayed on the display region 1381 in a superimposed manner, and the second image data 1392 and second light intensity distribution 1394 are displayed on the display region 1382 in a superimposed manner.

Next, as illustrated in Fig. 14, an example will be described wherein each image data and each light intensity distribution are displayed on a single display region in a superimposed manner.

First, the image data output module 253 outputs first image data 1491, second image data 1492, a first light intensity distribution 1493, and a second light intensity distribution 1494 to a single display region 1481. The first image data 1491, second image data 1492, first light intensity distribution 1493, and second light intensity distribution 1494 are then displayed on the display region 1481 in a superimposed manner.

Note that, with regard to display of a light intensity distribution as well, as with display of image data according to the first embodiment, it is desirable to enable transmittance, colors, shading, and so forth to be set. At this time, it is further desirable to set these using an operating unit so that a worker is allowed to perform operations in an interactive manner.

Also, the image data output module 253 may output, in the same way as with the light intensity distribution described in the present embodiment, a sensitivity distribution of the acoustic wave detector 240 to the display device 280 and causes the display device 280 to display this. An SN ratio of a detection signal corresponding to a region where the sensitivity of the acoustic wave detector 240 is high, and accordingly, a sensitivity distribution of the acoustic wave detector 240 is displayed on the display device in addition to image data, and accordingly, of the image data, a region where reliability is high obtained from a detection signal with a high SN ratio may be determined. Note that, with the present disclosure, the sensitivity of the acoustic wave detector mentioned here includes attenuation of acoustic waves in a propagation path from the sound source to the acoustic wave detector, and the directivity angle of the acoustic wave detector.

Third Embodiment

The present embodiment differs from other embodiments in that of a light intensity distribution, a confidence region which is a region where light is sufficiently irradiated is obtained. The confidence region mentioned here includes data to be displayed on the display unit obtained by performing luminance value conversion on the confidence region.

With the photoacoustic imaging, it is desirable that of the light intensity distribution obtained in the second embodiment, a region where light is sufficiently irradiated is displayed in an enhanced manner. Therefore, with the present embodiment, of a light intensity distribution, distinction property between optical absorber images and artifacts is further improved using a confidence region which is a region where light is sufficiently irradiated.

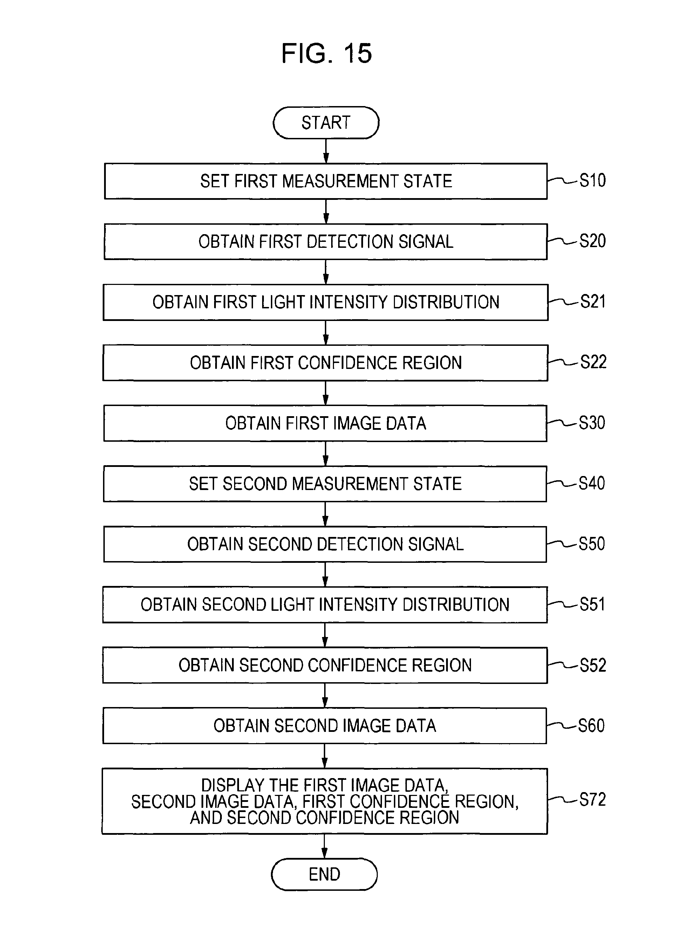

Hereinafter, a subject information obtaining method using a confidence region will be described with reference to the flowchart illustrated in Fig. 15. Note that the same processing as with the flowchart illustrated in Fig. 3 will be denoted with the same processing number, and description thereof will be omitted. Also, description will be made using the subject information obtaining device illustrated in Fig. 2.

S22, S52: Process to Obtain Confidence Region

First, the confidence region obtaining module 255 of the signal processing device 250 sets a threshold whereby it is conceived that a sufficient number of signals are obtained for each of the light intensity distributions.

Next, the confidence region obtaining module 255 performs processing to increase a light intensity value of a region of which the light intensity value is greater than the threshold, or processing to decrease a light intensity value of a region of which the light intensity value is smaller than the threshold, to obtain a confidence region with a region where light is sufficiently irradiated being enhanced. Specifically, the confidence region obtaining module 255 obtains a first confidence region based on the first light intensity distribution, and obtains a second confidence region based on the second light intensity distribution. Here, with regard to the threshold whereby a sufficient number of signals are obtained, it is desirable to set a desired level based on the SN ratio.

Note that the confidence region obtaining module 255 may obtain a confidence region by binarizing a light intensity distribution with the threshold as a reference.

Also, the confidence region obtaining module 255 may calculate the logic operation AND of the confidence region in each measurement state to take a result thereof as a confidence region.

S72: Process to Display Image Data and Confidence Region

Next, the image data output module 253 outputs the confidence region obtained in S22 or S52 to the display device 280, and causes the display device 280 to display the confidence region. With the present process, the confidence region may be displayed by being superimposed on the above- mentioned image data and light intensity distribution, or may be displayed in a row with the image data and light intensity distribution.

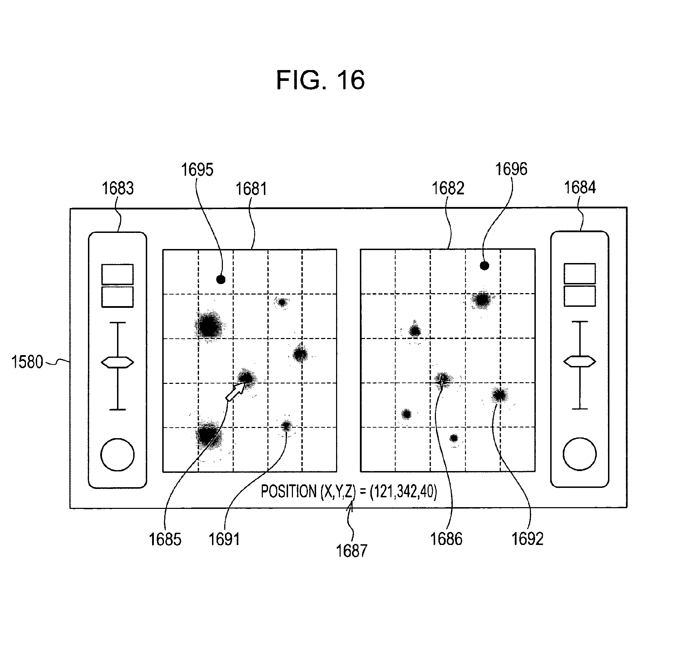

With the present embodiment, as an example thereof, an example illustrated in Fig. 16 will be described wherein a confidence region is displayed by being superimposed on the image data illustrated in Fig. 9.

First, the image data output module 253 outputs first image data 1691 and a first confidence region 1695 to a first display region 1681, and outputs second image data 1692 and a second confidence region 1696 to a second display region 1682. The first image data 1691 and first confidence region 1695 are superimposed and displayed on the first display region 1681, and the second image data 1692 and second confidence region 1696 are superimposed and displayed on the second display region 1682. Note that, in the event of superimposing and displaying the image data and confidence regions, it is desirable to display the image data and confidence regions by changing the transmittance of the image data or confidence regions. Also, it is desirable to display the image data and confidence regions with different colors. Further, with regard to the confidence regions, it is desirable to change their colors for each measurement state.

Also, in the event of having obtained confidence regions by binarizing a light intensity distribution, the outer circumferences of the confidence regions may be surrounded with a line. However, even in the event of having obtained confidence regions without binarizing, predetermined values in the confidence regions may be connected with a line to obtain the outer circumferences of the confidence regions.

In this manner, not only image data but also a confidence region where light is sufficiently irradiated are displayed, and accordingly, multiple image data with a region where reliability of image data in each measurement state is high may readily be compared.

Also, the confidence region obtaining module 255 may obtain, in the same way as with the confidence region based on a light intensity distribution described in the present embodiment, a confidence region based on the sensitivity distribution of the acoustic wave detector 240. In this case, the image data output module 253 may output the confidence region based on the sensitivity distribution of the acoustic wave detector 240 to the display device 280, and causes the display device 280 to display this.

The confidence region based on the sensitivity distribution of the acoustic wave detector 240 is image data where a region with high sensitivity of the acoustic wave detector 240 is enhanced. Therefore, the confidence region based on the sensitivity of the acoustic wave detector 240 also indicates, as with the confidence region based on a light intensity distribution, a region obtained from a detection signal with a high SN ratio.

Accordingly, in addition to image data, confidence regions based on the sensitivity distribution of the acoustic wave detector 240 are displayed on the display device 280, and accordingly, of multiple image data, a region with high reliability obtained from a detection signal with a high SN ratio may readily be compared.

Fourth Embodiment

With the present embodiment, it is difference with other embodiments to obtain image data based on a detection signal obtained by the acoustic wave detector and the confidence region obtained in the third embodiment.

With the present embodiment, the image data obtaining module 252 obtains image data where a region with high reliability is enhanced by weighing a confidence region for an optical characteristic value distribution obtained based on a detection signal.

Note that a detection signal in each measurement state may be weighed with a confidence region. In this case, signal intensity of detection time corresponding to a confidence region is weighed with the confidence region. Based on a detection signal weighed with the confidence region, image data where a region with high reliability is enhanced may be obtained.

However, in the event that confidence regions have been binarized, only signal intensity of detection time corresponding to a confidence region with a low value may be reduced. Also, even in the event that confidence regions have not been binarized, signal intensity of detection time corresponding to a confidence region with a predetermined value or less may be reduced.

Now, as a weighing method, a method to perform multiplication between an optical characteristic value distribution or similarity distribution and a confidence region may be employed, for example. Note that processing other than multiplication may be performed as long as a similarity distribution where a region with high reliability is enhanced may be obtained by the method.

Hereinafter, a subject information obtaining method according to the present embodiment will be described using the subject information obtaining device illustrated in Fig. 2.

For example, the image data obtaining module 252 obtains an initial sound pressure distribution by performing reconstitution processing on a first detection signal. The image data obtaining module 252 multiplies this initial sound pressure distribution, and a first confidence region based on a first light intensity distribution, thereby obtaining first image data.

In the same way, the image data obtaining module 252 multiplies a second detected signal, and a second confidence region based on a second light intensity distribution, thereby obtaining second image data.

The image data output module 253 displays the first image data and second image data so as to compare both using the display method described in the first embodiment.

With the image data thus displayed, optical characteristic values of a region where light intensity is sufficiently irradiated, or a region where the sensitivity of the acoustic wave detector is high are enhanced, and accordingly, optical characteristic values with high reliability may readily be determined. Therefore, optical characteristic values with high reliability are compared, and accordingly, distinction between optical absorber images and artifacts may readily be performed.

Fifth Embodiment

Majority of function information obtained by the photoacoustic imaging is information relating to an optical absorption coefficient, and accordingly, it is desirable to obtain information relating to an optical absorption coefficient as image data.

Therefore, with the present embodiment, information relating to an optical absorption coefficient distribution obtained by performing light intensity correction on an initial sound pressure distribution or optical energy density distribution is handled as image data. That is to say, with the present embodiment, examples of the image data include an optical absorption coefficient distribution and an oxygen saturation distribution.

Hereinafter, an example of a subject information obtaining method according to the present embodiment will be described with reference to the flowchart illustrated in Fig. 11. Note that, with regard to its configuration, description will be made with reference to the configuration of the subject information obtaining device illustrated in Fig. 2.

For example, in S30 according to the present embodiment, first, the image data obtaining module 252 obtains, based on Expression (1), a first optical absorption coefficient distribution serving as first image data using the first detection signal obtained in S20 and the first light intensity distribution obtained in S21. Also, similarly, in S60 according to the present embodiment, the image data obtaining module 252 obtains a second optical absorption coefficient distribution serving as second image data using the second detection signal obtained in S50 and the second light intensity distribution obtained in S51.

The image data output module 253 then outputs the first optical absorption coefficient distribution and second optical absorption coefficient distribution to the display device 280 so as to compare these, and the first optical absorption coefficient distribution and second optical absorption coefficient distribution are displayed on the display device 280.

With an optical absorption coefficient distribution with an initial sound pressure distribution thus subjected to light intensity correction, not only the position of an optical absorber image in each of the measurement states is the same, but also intensity of an optical absorber image is the same in each of the measurement states.

On the other hand, artifacts are failed to be removed even when performing light intensity correction thereon, and accordingly emerge in different positions depending on the measurement states.

Therefore, as with the present embodiment, image data based on information subjected to light intensity correction in each measurement state is displayed so as to be compared, and accordingly, an image existing in the same position and same intensity may be distinguished from optical absorber images. Accordingly, distinction between optical absorber images and artifacts may readily be distinguished.

Note that, as with oxygen saturation or the like, in the event of taking optical characteristic values obtained by multiple times of measurement as image data, multiple times of measurement for obtaining a first oxygen saturation distribution serving as first image data is taken as measurement in a first measurement state. An arrangement may be made wherein different multiple times of measurement is taken as measurement in a second measurement state, and a second oxygen saturation distribution serving as second image data is obtained. Here, different multiple times of measurement mentioned here indicates measurement wherein of multiple measurements, the measurement state of at least one measurement has been changed.

Hereinafter, the basic configuration of the subject information obtaining device illustrated in Fig. 2 will be described.

Light Source 210

The light source 210 is a device to generate pulsed light. In order to obtain large output, a laser is desirable as the light source 210, but may be a light-emitting diode or the like. In order to effectively generate photoacoustic waves, light has to be irradiated in a sufficiently short period of time according to a thermal property of the subject 230. In the event that the subject 230 is a living body, it is desirable to set 10 nanoseconds or less as the pulse width of pulsed light generated from the light source 210. Also, the wavelength of pulsed light is a near-infrared region called as a window of a living body, and is preferably around 700 nm to 1200 nm. Light within this region may reach a relatively deep portion of the living body, and accordingly, information of the deep portion may be obtained. Further, with regard to the wavelength of pulsed light, it is desirable that an optical absorption coefficient is high for an object to be observed.

Optical System 220

The optical system 220 is a device to guide pulsed light generated at the light source 210 to the subject 230. The optical system 220 is specifically an optical apparatus such as an optical fiber, lens, mirror, diffuser plate, or the like.

With the present disclosure, at the time of multiple times of measurement, the measurement state such as the irradiation shape of pulsed light, light density, irradiation direction for a subject, or the like may be changed using such an optical apparatus. Also, these may be adjusted at the light source 210.

Also, in order to obtain data in a wide range, the optical system 220 may be scanned by the optical scanning mechanism 221 configured so as to scan the optical system 220 to scan the irradiation position of pulsed light. At this time, scanning may be performed in conjunction with the acoustic wave detector 240.

Also, the optical system 220 is not restricted to that mentioned above, and any may be employed as long as this satisfies such a function.

Subject 230

The subject 230 becomes an object to be measured. As the subject 230, a living body or a phantom which has simulated a living body, or the like may be employed.

For example, in the event that the subject 230 is a living body, with the subject information obtaining device according to the present disclosure, a blood vessel or the like serving as an optical absorber existing within the subject 230 may be imaged. Also, examples of an optical absorber include hemoglobin, water, melanin, collagen, lipid, and so forth which have a relatively great optical absorber coefficient within a living body, and a living body tissue configured of these.

Also, in the event of a phantom, a substance which has simulated optical characteristics of the above-mentioned optical absorbers may be sealed in the phantom. Acoustic Wave Detector 240

The acoustic wave detector 240 detects photoacoustic waves, and converts these into electric signals.

In order to detect photoacoustic waves in multiple positions, the acoustic wave detector scanning mechanism 241 which is configured so as to scan the acoustic wave detector 240 may scan a single acoustic wave detector to move to multiple positions, or multiple acoustic wave detectors may be installed in different locations.

Also, with the photoacoustic imaging, the acoustic wave detector 240 receives photoacoustic waves generated from the inside of the subject 230, and accordingly, in order to suppress reflection or attenuation of generated photoacoustic waves, the acoustic wave detector 240 has to be installed so as to acoustically be connected to the subject 230. For example, an acoustic matching material such as acoustic matching GEL, water, oil, or the like may be provided between the acoustic wave detector 240 and the subject 230.

Also, as the acoustic wave detector 240, a device with high sensitivity and wide frequency band is desirable, and specific examples thereof include a PZT, PVDF, cMUT, and acoustic wave detector using a Fabr-Perot interferometer. However, the acoustic wave detector 240 is not restricted to mentioned above, and any may be employed as long as this satisfies the function.

Signal Processing Device 250

The signal processing device 250 performs amplification or conversion into digital signals, or the like regarding electric signals obtained at the acoustic wave detector 240. The converted digital signals are then processed to obtain image data, and the image data is output to the display device 280. The signal processing device 250 includes an AD converter (ADC), the measurement state setting module 251, image data obtaining module 252, image data output module 253, light intensity distribution obtaining module 254, confidence region obtaining module 255, and so forth.

Note that the modules which the signal processing device 250 according to the present embodiment includes may be provided as stand-alone configurations, respectively.

Also, in the event of configuring the modules as hardware, the modules are able to be configured as FPGA, ASIC, or the like. Also, each of the modules may be configured as a program causing the computer to execute each of the processes.

Specific examples of the signal processing device include a computer and an electric circuit. In order to effectively obtain data, it is desirable to include AD converters (ADC) of which the number is the same as the number of reception elements of the acoustic wave detector 240, but one ADC may be used by sequentially changing a reception element to be connected.

Memory 260

The memory 260 is configured to hold image data processed by the signal processing device 250. Image data obtained in a different measurement state is held in the memory. It is desirable to prepare memory by the number of measurement states.

Note that, though the memory is to temporarily hold image data, and to enable a flexible display method to be performed, the signal processing device 250 may directly transmit image data to the display device 280 without employing the memory.

Note that the signal processing device 250 may include the memory 260, or the display device 280 may include the memory 260.

Display Device 280

The display device 280 includes a display region where image data is displayed. Also, the display device 280 may include multiple display regions. Note that, with the present disclosure, the display unit means a single display device or multiple display devices.

Also, it is desirable that the display device 280 includes an operating unit to be used for operating a display state or pointer. Further, it is desirable that the single operating unit is provided to each of the display regions. Also, the operating unit may be operated by touch panel operations or by hardware operations such as a mechanical switch or the like. Note that the operating unit may be provided to a device other than the display device 280, e.g., the signal processing device 250. Also, the operating unit may be a stand-alone device.

Also, though it is desirable that the first display region and second display region are displayed in the same location, same range, and same dynamic range in an interlocking manner, the display regions may individually be adjusted. Image processing used for such adjustment may be performed by the image data output module 253.

Also, the signal processing device 250 and display device 280 may be provided in an integral manner.

Other Embodiments

Embodiments of the present invention can also be realized by a computer of a system or apparatus that reads out and executes computer executable instructions recorded on a storage medium (e.g., non-transitory computer-readable storage medium) to perform the functions of one or more of the above-described embodiment(s) of the present invention, and by a method performed by the computer of the system or apparatus by, for example, reading out and executing the computer executable instructions from the storage medium to perform the functions of one or more of the above-described embodiment(s). The computer may comprise one or more of a central processing unit (CPU), micro processing unit (MPU), or other circuitry, and may include a network of separate computers or separate computer processors. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

**Claims**

1. A subject information obtaining device comprising:

first image data obtaining means (252) arranged to obtain first image data based on a first detection signal obtained by detecting first photoacoustic waves generated by irradiating light in a first measurement state on a subject (130, 131); second image data obtaining means (252) arranged to obtain second image data based on a second detection signal obtained by detecting second photoacoustic waves generated by irradiating light in a second measurement state different from the first measurement state on the subject (130, 131); and image data output means (253) arranged to display the first image data and the second image data on display means (280) so as to compare the first image data and the second image data at the display means (280).

2. The subject information obtaining device according to Claim 1, wherein the image data output means (253) cause the display means (280) to execute any one of parallel display, superimposed display, and alternating display regarding the first image data and the second image data.

3. The subject information obtaining device according to either of Claims 1 or 2, further comprising:

a light intensity distribution obtaining means (254) arranged to obtain a first light intensity distribution within the subject (130, 131) when irradiating light in the first measurement state on a subject (130, 131), and a second light intensity distribution within the subject (130, 131) when irradiating light in the second measurement state on the subject (130, 131).

4. The subject information obtaining device according to Claim 3, wherein the image data output means (253) cause the display means (280) to display the first light intensity distribution and the second light intensity distribution.

5. The subject information obtaining device according to either of Claims 3 or 4, wherein the first image data obtaining means (252) obtain the first image data based on the first detection signal and the first light intensity distribution; and wherein the second image data obtaining means (252) obtain the second image data based on the second detection signal and the second light intensity distribution.

6. The subject information obtaining device according to any one of Claims 3 through 5, further comprising:

a confidence region obtaining means (255) arranged to obtain a first confidence region based on the first light intensity distribution or a second confidence region based on the second light intensity distribution by performing processing to set a threshold for the first light intensity distribution or the second light intensity distribution to increase a light intensity value of a region of which the light intensity is greater than the threshold, or processing to decrease a light intensity value of a region of which the light intensity is smaller than the threshold; wherein the image data output means (253) cause the display means (280) to display the first confidence region or the second confidence region.

7. The subject information obtaining device according to any one of Claims 3 through 5, further comprising:

confidence region obtaining means (255) arranged to obtain a first confidence region based on the first light intensity distribution or a second confidence region based on the second light intensity distribution by performing processing to set a threshold for the first light intensity distribution or the second light intensity distribution to increase a light intensity value of a region of which the light intensity is greater than the threshold, or processing to decrease a light intensity value of a region of which the light intensity is smaller than the threshold; wherein the first image data obtaining means (252) obtain the first image data based on the first detection signal and the first confidence region; and wherein the second image data obtaining means (252) obtain the second image data based on the second detection signal and the second confidence region.

8. The subject information obtaining device according to any one of Claims 1 through 7, wherein the image data output means (253) cause the display means (280) to display the first image data on a first display region of the display means (280), and cause the display means (280) to display the second image data on a second display region of the display means (280) which differs from the first image region.

9. The subject information obtaining device according to any one of Claims 3 through 7, wherein the image data output means (253) cause the display means (280) to display the first image data and the first light intensity distribution on a first display region of the display means (280) in a superimposed manner, and cause the display means (280) to display the second image data and the second light intensity distribution on a second display region of the display means (280) which differs from the first display region, in a superimposed manner.

10. The subject information obtaining device according to either of Claims 6 or 7, wherein the image data output means (253) cause the display means (280) to display the first image data and the first confidence region on a first display region of the display means (280) in a superimposed manner, and cause the display means (280) to display the second image data and the second confidence region on a second display region of the display means (280) which differs from the first display region, in a superimposed manner.

11. The subject information obtaining device according to any one of Claims 1 through 7, wherein the image data output means (253) cause the display means (280) to display the first image data and the second image data in a superimposed manner.

12. The subject information obtaining device according to any one of Claims 3 through 7, wherein the image data output means (253) cause the display means (280) to display the first image data, the second image data, the first light intensity distribution, and the second light intensity distribution in a superimposed manner.

13. The subject information obtaining device according to any one of Claims 1 through 12, wherein the first measurement state and the second measurement state include any one of:

irradiation positions of light in the first measurement state and light in the second measurement state for a surface of the subject (130, 131); angles made up of a surface of the subject (130, 131), and irradiation directions of light in the first measurement state and light in the second measurement state; irradiation intensities of light in the first measurement state and light in the second measurement state for a surface of the subject (130, 131); and positions of detection surfaces of an acoustic wave detector (140) arranged to detect the first photoacoustic waves and the second photoacoustic waves.

14. The subject information obtaining device according to any one of Claims 1 through 13, further comprising:

    measurement state setting means (251) arranged to set the first measurement state and the second measurement state.

15. The subject information obtaining device according to any one of Claims 1 through 14, further comprising:

    a light source (210) arranged to generate light;
an optical system (220) arranged to emit the light on the subject (130, 131) as light in the first measurement state and light in the second measurement state to generate the first photoacoustic waves and the second photoacoustic waves; and
an acoustic wave detector (140) arranged to detect the first photoacoustic waves and the second photoacoustic waves to output the first detection signal and the second detection signal.

16. A subject information obtaining method comprising:

    a process (S30) to obtain first image data based on a first detection signal obtained by detecting (S20) first photoacoustic waves generated by irradiating light (S10) in a first measurement state on a subject (130, 131);
a process (S60) to obtain second image data based on a second detection signal obtained by detecting (S50) second photoacoustic waves generated by irradiating light (S40) in a second measurement state different from the first measurement state on the subject (130, 131); and
a process (S70) to display the first image data and the second image data so as to compare these.

17. The subject (130, 131) information obtaining method according to Claim 16, further comprising:

    a process (S10) to irradiate light in the first measurement state on the subject (130, 131);
a process (S20) to obtain the first detection signal by detecting the first photoacoustic waves generated by irradiating light (S10) in the first measurement state on the subject (130, 131);
a process (S40) to irradiate light in the second measurement state on the subject (130, 131); and
a process (S50) to obtain the second detection signal by detecting the second photoacoustic waves generated by irradiating light (S40) in the second measurement state on the subject (130, 131).

18. A program causing a computer to execute the subject information obtaining method according to either of Claims 16 or 17.

FIG. 1A

FIG. 1B

FIG. 1C

FIG. 1D

# FIG. 2

LIGHT SOURCE —210

ACOUSTIC WAVE DETECTOR SCANNING MECHANISM —241

280

230

OPTICAL SYSTEM

220

SUBJECT

ACOUSTIC WAVE DETECTOR —240

DISPLAY DEVICE

OPTICAL SCANNING MECHANISM

221

251— MEASUREMENT STATE SETTING MODULE

IMAGE DATA OBTAINING MODULE —252

MEMORY

253— IMAGE DATA OUTPUT MODULE

LIGHT INTENSITY DISTRIBUTION OBTAINING MODULE —254

260

255— CONFIDENCE REGION OBTAINING MODULE

SIGNAL PROCESSING DEVICE —250

EP 2 638 851 A1

# FIG. 3

```
            ( START )
                |
   SET FIRST MEASUREMENT STATE       ── S10
                |
   OBTAIN FIRST DETECTION SIGNAL     ── S20
                |
   OBTAIN FIRST IMAGE DATA           ── S30
                |
   SET SECOND MEASUREMENT STATE      ── S40
                |
   OBTAIN SECOND DETECTION SIGNAL    ── S50
                |
   OBTAIN SECOND IMAGE DATA          ── S60
                |
   DISPLAY THE FIRST IMAGE DATA AND  ── S70
   SECOND IMAGE DATA FOR COMPARISON
                |
            ( END )
```

# FIG. 4A

# FIG. 4B

# FIG. 5A

# FIG. 5B

## FIG. 6A

630
645
625
620
640

## FIG. 6B

631
646
626
621
641

## FIG. 7A

726
730
745
721
725
720
740

## FIG. 7B

728
731
746
723
727
722
741

# FIG. 8A

# FIG. 8B

# FIG. 9

POSITION (X,Y,Z) = (121,342,40)

# FIG. 10

# FIG. 11

```
                    ┌─────────────┐
                    │    START    │
                    └──────┬──────┘
                           │
                           ▼
        ┌──────────────────────────────────────┐
        │     SET FIRST MEASUREMENT STATE       │─── S10
        └──────────────────┬───────────────────┘
                           │
                           ▼
        ┌──────────────────────────────────────┐
        │      OBTAIN FIRST DETECTION SIGNAL     │─── S20
        └──────────────────┬───────────────────┘
                           │
                           ▼
        ┌──────────────────────────────────────┐
        │  OBTAIN FIRST LIGHT INTENSITY DISTRIBUTION │─── S21
        └──────────────────┬───────────────────┘
                           │
                           ▼
        ┌──────────────────────────────────────┐
        │        OBTAIN FIRST IMAGE DATA         │─── S30
        └──────────────────┬───────────────────┘
                           │
                           ▼
        ┌──────────────────────────────────────┐
        │     SET SECOND MEASUREMENT STATE       │─── S40
        └──────────────────┬───────────────────┘
                           │
                           ▼
        ┌──────────────────────────────────────┐
        │     OBTAIN SECOND DETECTION SIGNAL     │─── S50
        └──────────────────┬───────────────────┘
                           │
                           ▼
        ┌──────────────────────────────────────┐
        │ OBTAIN SECOND LIGHT INTENSITY DISTRIBUTION │─── S51
        └──────────────────┬───────────────────┘
                           │
                           ▼
        ┌──────────────────────────────────────┐
        │       OBTAIN SECOND IMAGE DATA         │─── S60
        └──────────────────┬───────────────────┘
                           │
                           ▼
        ┌──────────────────────────────────────┐
        │ DISPLAY THE FIRST IMAGE DATA, SECOND   │
        │ IMAGE DATA, FIRST LIGHT INTENSITY      │─── S71
        │ DISTRIBUTION, AND SECOND LIGHT         │
        │ INTENSITY DISTRIBUTION                 │
        └──────────────────┬───────────────────┘
                           │
                           ▼
                    ┌─────────────┐
                    │     END     │
                    └─────────────┘
```

# FIG. 12

POSITION (X,Y,Z) = (121,342,40)

## FIG. 13

POSITION (X,Y,Z) = (121,342,40)

## FIG. 14

# FIG. 15

```
          ┌─────────┐
          │  START  │
          └─────────┘
               │
               ▼
┌───────────────────────────────────────┐
│        SET FIRST MEASUREMENT STATE     │───S10
└───────────────────────────────────────┘
               │
               ▼
┌───────────────────────────────────────┐
│       OBTAIN FIRST DETECTION SIGNAL    │───S20
└───────────────────────────────────────┘
               │
               ▼
┌───────────────────────────────────────┐
│    OBTAIN FIRST LIGHT INTENSITY DISTRIBUTION │───S21
└───────────────────────────────────────┘
               │
               ▼
┌───────────────────────────────────────┐
│      OBTAIN FIRST CONFIDENCE REGION    │───S22
└───────────────────────────────────────┘
               │
               ▼
┌───────────────────────────────────────┐
│         OBTAIN FIRST IMAGE DATA        │───S30
└───────────────────────────────────────┘
               │
               ▼
┌───────────────────────────────────────┐
│       SET SECOND MEASUREMENT STATE     │───S40
└───────────────────────────────────────┘
               │
               ▼
┌───────────────────────────────────────┐
│      OBTAIN SECOND DETECTION SIGNAL    │───S50
└───────────────────────────────────────┘
               │
               ▼
┌───────────────────────────────────────┐
│  OBTAIN SECOND LIGHT INTENSITY DISTRIBUTION │───S51
└───────────────────────────────────────┘
               │
               ▼
┌───────────────────────────────────────┐
│     OBTAIN SECOND CONFIDENCE REGION    │───S52
└───────────────────────────────────────┘
               │
               ▼
┌───────────────────────────────────────┐
│        OBTAIN SECOND IMAGE DATA        │───S60
└───────────────────────────────────────┘
               │
               ▼
┌───────────────────────────────────────┐
│         DISPLAY THE FIRST IMAGE DATA,  │
│ SECOND IMAGE DATA, FIRST CONFIDENCE REGION, │───S72
│      AND SECOND CONFIDENCE REGION      │
└───────────────────────────────────────┘
               │
               ▼
          ┌─────────┐
          │   END   │
          └─────────┘
```

FIG. 16

POSITION (X,Y,Z) = (121,342,40)

Europäisches
Patentamt

European
Patent Office

Office européen
des brevets

**EUROPEAN SEARCH REPORT**

Application Number

EP 13 00 0839

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/004458 A1 (KANAYAMA SHOICHI [JP] ET AL) 6 January 2005 (2005-01-06) | 1,2,8, 11,13-18 | INV. A61B5/00 |
| Y | * abstract * <br> * paragraphs [0076], [0096] * <br> * paragraphs [0107] - [0118] * <br> * paragraph [0206] * | 3-7,9, 10,12 | G01N21/17 |
| Y | WO 2011/122382 A1 (CANON KK [JP]; MIYASATO TAKURO [JP]) 6 October 2011 (2011-10-06) <br> * abstract * <br> * paragraphs [0025], [0026], [0038] * | 3-7,9, 10,12 | |
| X | US 5 713 356 A (KRUGER ROBERT A [US]) 3 February 1998 (1998-02-03) <br> * abstract * <br> * column 9, lines 10-35 * | 1,2, 15-18 | |
| X | JO JANGGUN ET AL: "Photoacoustic imaging to detect rat brain activation after cocaine hydrochloride injection", PHOTONS PLUS ULTRASOUND: IMAGING AND SENSING 2011, SPIE, 1000 20TH ST. BELLINGHAM WA 98225-6705 USA, vol. 7899, no. 1, 10 February 2011 (2011-02-10), pages 1-8, XP060007477, DOI: 10.1117/12.875670 [retrieved on 2011-02-22] <br> * abstract * <br> * figure 4 * <br> * Item "2.1. Photoacoustic imaging system". * | 1,2,8, 11,13-18 | |
| X | US 2011/232385 A1 (NANAUMI RYUICHI [JP]) 29 September 2011 (2011-09-29) | 16-18 | |
| A | * paragraphs [0026] - [0038] * | 1-15 | |

-/--

TECHNICAL FIELDS SEARCHED (IPC)

A61B
G01N

The present search report has been drawn up for all claims

| Place of search | Date of completion of the search | Examiner |
|---|---|---|
| The Hague | 2 July 2013 | Dhervé, Gwenaëlle |

**EP 2 638 851 A1**

<table>
<tr><td rowspan="2">Europäisches<br>Patentamt<br>European<br>Patent Office<br>Office européen<br>des brevets</td><td rowspan="2">**EUROPEAN SEARCH REPORT**</td><td>Application Number</td></tr>
<tr><td>EP 13 00 0839</td></tr>
</table>

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2010/049049 A1 (ASAO YASUFUMI [JP] ET AL) 25 February 2010 (2010-02-25) | 16-18 | |
| A | * paragraphs [0047] - [0064], [0076] *<br>----- | 1-15 | |
| | | | **TECHNICAL FIELDS SEARCHED    (IPC)** |

The present search report has been drawn up for all claims

| Place of search | Date of completion of the search | Examiner |
|---|---|---|
| The Hague | 2 July 2013 | Dhervé, Gwenaëlle |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 13 00 0839

This annex lists the patent family members relating to the patent documents cited in the above-mentioned European search report.
The members are as contained in the European Patent Office EDP file on
The European Patent Office is in no way liable for these particulars which are merely given for the purpose of information.

02-07-2013

| Patent document cited in search report | | Publication date | Patent family member(s) | | Publication date |
|---|---|---|---|---|---|
| US 2005004458 | A1 | 06-01-2005 | CA | 2435990 A1 | 02-01-2005 |
| | | | CN | 1575770 A | 09-02-2005 |
| | | | EP | 1493380 A1 | 05-01-2005 |
| | | | JP | 4406226 B2 | 27-01-2010 |
| | | | JP | 2005021380 A | 27-01-2005 |
| | | | KR | 20050003948 A | 12-01-2005 |
| | | | KR | 20060080562 A | 10-07-2006 |
| | | | US | 2005004458 A1 | 06-01-2005 |
| WO 2011122382 | A1 | 06-10-2011 | CN | 102843960 A | 26-12-2012 |
| | | | EP | 2552299 A1 | 06-02-2013 |
| | | | JP | 2011206192 A | 20-10-2011 |
| | | | KR | 20120126109 A | 20-11-2012 |
| | | | US | 2013006088 A1 | 03-01-2013 |
| | | | WO | 2011122382 A1 | 06-10-2011 |
| US 5713356 | A | 03-02-1998 | AT | 408374 T | 15-10-2008 |
| | | | AU | 725072 B2 | 05-10-2000 |
| | | | BR | 9712262 A | 25-01-2000 |
| | | | CA | 2187701 A1 | 04-04-1998 |
| | | | EP | 0942683 A1 | 22-09-1999 |
| | | | JP | 4341987 B2 | 14-10-2009 |
| | | | JP | 2001507952 A | 19-06-2001 |
| | | | US | 5713356 A | 03-02-1998 |
| | | | US | 6102857 A | 15-08-2000 |
| | | | US | 6292682 B1 | 18-09-2001 |
| | | | US | 2002035327 A1 | 21-03-2002 |
| | | | WO | 9814118 A1 | 09-04-1998 |
| US 2011232385 | A1 | 29-09-2011 | JP | 2011200414 A | 13-10-2011 |
| | | | US | 2011232385 A1 | 29-09-2011 |
| US 2010049049 | A1 | 25-02-2010 | JP | 2010046215 A | 04-03-2010 |
| | | | US | 2010049049 A1 | 25-02-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2010035806 A

**Non-patent literature cited in the description**

- **MINGHUA XU ; LIHONG V. WANG.** Universal back-projection algorithm for photoacoustic computed tomography. *PHYSICAL REVIEW,* 2005, vol. E 71, 016706